# EUROPÄISCHE PATENTSCHRIFT

(11) **EP 4 009 920 B1**
(45) Veröffentlichungstag und Bekanntmachung des Hinweises auf die Patenterteilung: **09.07.2025**
(21) Anmeldenummer: 20745132.9
(22) Anmeldetag: 21.07.2020
(51) Int. Cl.: A61F 2/90, A61B 17/12, A61F 2/82

(54) **MEDIZINISCHES SET ZUR BEHANDLUNG VON ANEURYSMEN, HERSTELLUNGSVERFAHREN SOWIE MEDIZINISCHES SYSTEM ZUR BEHANDLUNG VON ANEURYSMEN**
MEDICAL SET FOR TREATING ANEURYSMS, PRODUCTION PROCESS, AND MEDICAL SYSTEM FOR TREATING ANEURYSMS
ENSEMBLE MÉDICAL POUR TRAITER DES ANÉVRISMES, PROCÉDÉ DE FABRICATION ET SYSTÈME MÉDICAL POUR TRAITER DES ANÉVRISMES

(30) Priorität: 09.08.2019 DE 102019121554
(43) Veröffentlichungstag der Anmeldung: 15.06.2022
(73) Patentinhaber: Acandis GmbH, 75177 Pforzheim (DE)
(72) Erfinder: BÜCHERT, Michael, 75177 Pforzheim (DE); SCHÖNBERGER, Eugen, 75176 Pforzheim (DE); CATTANEO, Giorgio, 76199 Karlsruhe (DE)
(74) Vertreter: Meissner Bolte Partnerschaft mbB
(86) Internationale Anmeldenummer: PCT/EP2020/070500
(87) Internationale Veröffentlichungsnummer: WO 2021/028161

(56) Entgegenhaltungen:
- EP-A1- 2 505 149
- US-A1- 2018 193 026

## Beschreibung

Die Erfindung betrifft ein medizinisches Set zur Behandlung von Aneurysmen nach dem Oberbegriff des Patentanspruchs 1. Ferner betrifft die Erfindung ein Herstellungsverfahren nach Anspruch 11 sowie ein medizinisches System nach Anspruch 12.

WO 2014/177634 A1 beschreibt einen hochflexiblen Stent, der eine komprimierbare und expandierbare Gitterstruktur aufweist, wobei die Gitterstruktur einstückig ausgebildet ist. Die Gitterstruktur umfasst geschlossene Zellen, die durch jeweils vier Gitterelemente begrenzt sind. Die Gitterstruktur weist wenigstens einen Zellenring auf, der zwischen drei und sechs Zellen umfasst.

US 2018/0193026 A1 befasst sich mit einer Vorrichtung zur Abdeckung von Aneurysmen mit einer Gitterstruktur, die über einen Katheter an den Behandlungsort transportierbar ist. Die Gitterstruktur ist mit einer Umhüllung versehen, die auch die Öffnungen der Gitterstruktur bedeckt und so das Aneurysma vom Blutfluss abtrennt. Die Umhüllung schränkt die Komprimierbarkeit der Gitterstruktur ein und birgt das Risiko zu reißen, wenn die Gitterstruktur starken Verformungen ausgesetzt ist.

Aus der Praxis der Anmelderin sind außerdem Stents mit Gitterstrukturen bekannt, die aus einem einzigen Draht gebildet sind. Der Draht ist mit sich selbst verflochten, um ein röhrchenförmiges Geflecht zu bilden. An den axialen Enden des röhrchenförmigen Geflechts ist der Draht umgelenkt, so dass sich atraumatisch wirkende Schlaufen bilden. Die axialen Enden können trichterförmig ausgeweitet sein.

Die bekannte medizinische Vorrichtung eignet sich insbesondere zur Behandlung von Aneurysmen in kleinen, zerebralen Blutgefäßen. Derartige Blutgefäße weisen einen sehr kleinen Querschnittsdurchmesser auf und sind oft stark gewunden. Der bekannte Stent ist dazu hochflexibel gestaltet, so dass er einerseits auf einen sehr kleinen Querschnittsdurchmesser komprimierbar ist und andererseits eine hohe Biegeflexibilität aufweist, die die Zuführung in kleine zerebrale Blutgefäße ermöglicht.

Zur Behandlung von Aneurysmen in zerebralen Blutgefäßen ist es zweckmäßig, Stents einzusetzen, die sich über ein Aneurysma spannen und dieses vom Blutfluss innerhalb des Blutgefäßes abschirmen. Um dies zu ermöglichen, ist es bekannt, Stents mit einer Abdeckung zu versehen, die die Zellen des Stents verschließt und so einen Blutstrom in ein Aneurysma sowie eine Perfusion von Seitenästen des Blutgefäßes verhindert.

Eine weitere ergänzende oder alternative Behandlungsmethode von Aneurysmen ist die Implantation sogenannter Coils in das Aneurysma, welche dort zu einer Blutgerinnung führt. Der resultierende Thrombus verhindert dann die Blutzirkulation im Aneurysma und somit das Risiko einer Ruptur mit anschließender Blutung.

Besonders bei breithalsigen Aneurysmen neigen die Coils jedoch dazu, während der Implantation in die Blutbahn zu wandern und somit einen Verschluss des Hauptgefäßlumen zu verursachen. Bei der Technik "Ballon assisted Coiling" werden Katheter mit "Compliance-Ballonen" im Gefäß, speziell an der Höhe des Aneurysmahalses, positioniert. Der mit Kontrastmittel gefüllte Ballon schließt den Aneurysmahals während der Platzierung der Coils und zwingt die Coils in eine kompakte Anordnung innerhalb des Aneurysma-Raums. Weil Coils plastisch verformbar sind, bleiben diese dann in ihrer Form. Sie verlassen das Aneurysma auch dann nicht, wenn der Ballon entfernt wird. Ein Problem besteht jedoch hierbei insbesondere aufgrund der Tatsache, dass der Ballon das Gefäß verschließt. Bei anhaltender Prozedur (bei großen Aneurysmen werden mehrere Coils platziert, die Prozedur kann mehrere Minuten dauern), wird die Blutströmung in der Zeit vollständig unterbrochen. Zwar sorgen Kollateralgefäße für eine Versorgung von nachgelagertem Gewebe, trotzdem bleibt die Gefahr einer Unterperfusion bestehen.

Weiterhin wird während der Prozedur ein Katheter, durch den die Coils geführt werden, an der Seite des Ballons "gejailt", das heißt festgeklemmt. Bei der evtl. Notwendigkeit, Coil-Katheter zu wechseln (z.B im Fall einer Beschädigung) bei noch nicht vollständiger Prozedur, soll der Ballon deflatiert (entleert) werden, um den Katheter zurückziehen zu können. In dieser Phase kann es zu einer Verschiebung der Coils, die noch nicht vollständig und kompakt in dem Gefäß liegen, kommen. Diese können dann zu einem Gefäßverschluss führen.

Vor diesem Hintergrund ist es Aufgabe der Erfindung, ein medizinisches Set zur Behandlung von Aneurysmen anzugeben, mit dessen Hilfe die Gefahr eines Verschlusses von Seitenästen eines Blutgefäßeszumindest reduziert ist. Eine weitere Aufgabe der Erfindung besteht darin, ein Herstellungsverfahren für ein medizinisches Set sowie ein medizinisches System anzugeben.

Erfindungsgemäß wird diese Aufgabe im Hinblick auf das medizinische Set durch den Gegenstand des Patentanspruchs 1, im Hinblick auf das Verfahren zur Herstellung eines medizinischen Sets durch den Gegenstand des Patentanspruchs 11 und im Hinblick auf das medizinische System durch den Gegenstand des Patentanspruchs 12 gelöst.

Bevorzugte Ausgestaltungen, Weiterbildungen und Varianten sind Gegenstand der Unteransprüche.

Konkret wird Aufgabe gelöst durch ein medizinisches Set zur Behandlung von Aneurysmen mit einem Hauptkatheter sowie mit einer durch den Hauptkatheter hindurch an einen Behandlungsort bewegbaren Abdeckvorrichtung. Unter dem Behandlungsort kann hierbei eine Stelle entlang eines Gefäßes verstanden werden, an der das Aneurysma ausgebildet ist. Die Abdeckvorrichtung dient hierbei einem Abdecken des Aneurysmas. Ferner kann es sich bei dem Abdecken um ein temporäres Abdecken des Aneurysmas, beispielsweise lediglich für eine Dauer des Platzierens eines Embolisationsmittels innerhalb des Aneurysmas handeln. Besonders bevorzugt handelt es sich bei dem Abdecken um dauerhaftes, also permanentes Abdecken, beispielsweise in Form einer Dauerimplantation der Abdeckvorrichtung in das Gefäß, speziell in Form eines Stents oder eines Flow Diverters.

Die Abdeckvorrichtung kann mit einem Transportdraht mechanisch dauerfest, also unlösbar, verbunden sein. Falls die Abdeckvorrichtung ein Dauerimplantat, beispielsweise einen Stent oder einen Flow Diverter bildet, kann die Verbindung mit dem Transportdraht auch mechanisch ablösbar sein. Weiterhin weist die Abdeckvorrichtung eine selbstexpandierbare Gitterstruktur aus Stegen auf. Unter selbstexpandierbar kann hierbei verstanden werden, dass die Gitterstruktur von einem komprimierten Zustand in einem expandierten Zustand ohne eine Einwirkung von externen Kräften übergehen kann. Hierzu weist die Gitterstruktur vorzugsweise ein Formgedächtnismaterial auf oder ist aus einem derartigen Material gebildet.

Die Stege der Gitterstruktur sind einstückig miteinander verbunden und begrenzen Innenzellen sowie Randzellen, wobei die Randzellen an einem Längsende der Gitterstruktur, beispielsweise an einem distalen und/oder proximalen Längsende der Gitterstruktur, einen geschlossenen Randzellenring bilden. Der Randzellenring ist hierbei nur einseitig mit den Innenzellen verbunden. Unter dem Randzellenring kann hierbei ein Ring aus in Umfangsrichtung benachbarten Zellen, insbesondere Randzellen, verstanden werden.

Weiterhin ist die Gitterstruktur mit einer Abdeckung aus einem Gewebe versehen. Das Gewebe weist hierbei unregelmäßig große Poren auf. Wenigstens eine Innenzelle der Gitterstruktur ist dabei zumindest teilweise und insbesondere größtenteils abdeckungsfrei, d.h. sie ist nicht mit der Abdeckung oder mit einem Teil der Abdeckung versehen. Unter größtenteils abdeckungsfrei kann hierbei verstanden werden, dass eine insbesondere durchgehende Fläche von mehr als der Hälfte der Gesamtfläche der Gitterstruktur abdeckungsfrei ist.

Der Vorteil hierbei ist, dass zum einen durch die längsaxiale Blutdurchströmbarkeit der Gitterstruktur eine Blutströmung im Gefäß beispielsweise während des Setzens eines Coils gewährleistet ist. Zum anderen ist eine zuverlässige und speziell ortsspezifische Abdeckung des Aneurysmas sichergestellt, ohne jedoch Seitenäste durch die Abdeckung abzudecken. Ein Weiterer Vorteil ist in der Reduzierung der Gesamtfläche der Abdeckung zu sehen.

Wegen der längsaxial durchströmbaren Gitterstruktur wird somit ein Blutfluss, insbesondere in Längsrichtung durch das Blutgefäß, kaum behindert, jedoch eine Einströmung des Blutes in ein abzweigendes Aneurysma durch die Abdeckung unterbunden oder zumindest ein Strömungseinfluss in das Aneurysma reduziert. Besonders bei einer Dauerimplantation der Abdeckvorrichtung hat sich diese längsaxiale Durchströmbarkeit der Abdeckungsvorrichtung als besonders geeignet erwiesen. Ein mögliches und unerwünschtes Verschließen des Gefäßes, wie es beispielsweise bei der eingangs genannten Technik mittels des Ballons auftreten kann, ist somit zumindest reduziert und vorzugsweise ausgeschlossen. Durch die wenigstens eine Innenzelle, welche abdeckungsfrei ist, ist weiterhin eine Versorgung des dahinterliegenden Gefäßgewebes ermöglicht.

Eine solche Gestaltung der Gitterstruktur ermöglicht auch den Einsatz des medizinischen Sets als Stent bzw. Flow Diverter, der einen Blutfluss in Längsrichtung und auch z.B. zu Seitengefäßen durch das Blutgefäß kaum behindert, jedoch eine Einströmung des Blutes in ein abzweigendes Aneurysma durch die Abdeckung unterbindet oder zumindest den Strömungseinfluss reduziert. Ein mögliches und unerwünschtes Verschließen des Gefäßes, wie es beispielsweise bei der eingangs genannten Technik mittels des Ballons auftreten kann, ist somit zumindest reduziert und vorzugsweise ausgeschlossen. Weiterhin sind beispielsweise neben oder in einem Nahbereich des Aneurysmas befindliche Seitengefäße weiterhin blutdurchströmbar.

Insbesondere bei fusiformen Aneurysmen, d.h. Aneurysmen, die sich über den gesamten Umfang eines Blutgefäßes erstrecken, ist es vorteilhaft, eine gezielt feinporige Struktur zur Ansiedlung von Endothelzellen einzusetzen. Damit kann eine Rekonstruktion der fehlenden Gefäßwand erzielt werden. Konkret bildet die mit einer bestimmten Porengröße versehende Struktur, die durch das elektrogesponnene Gewebe gebildet ist, ein Gerüst für die Ansiedlung von Endothelzellen, die daraufhin eine neue, geschlossene Gefäßwand bilden können. Unter fusiformen Aneurysmen werden hierbei Aneurysmen verstanden, die sich über wenigstens 50%, insbesondere über wenigstens 75% des gesamten Umfangs oder über den gesamten Umfang eines Blutgefäßes erstrecken.

In einer bevorzugten Ausführungsform ist die Abdeckung aus einem elektrogesponnenen Gewebe gebildet.

Bei einem elektrogesponnenen Gewebe sind Poren üblicherweise unregelmäßig gestaltet. Das Herstellungsverfahren erlaubt es jedenfalls nicht, eine musterartige Anordnung oder Gestaltung von Poren zu erstellen. Allerdings können die Porengrößen anhand der Prozessparameter zumindest soweit eingestellt werden, dass sichergestellt ist, dass wenigstens ein Teil der Poren eine gewisse Mindestgröße aufweist.

Beispielsweise kann der Prozess des Elektrospinnens unmittelbar auf der Gitterstruktur erfolgen, so dass bei der Bildung der Abdeckung gleichzeitig eine Verbindung mit der Gitterstruktur hergestellt wird. Die Abdeckung kann mit der Gitterstruktur stoffschlüssig verbunden sein. Beispielsweise kann die Abdeckung mit der Gitterstruktur durch eine Klebeverbindung verbunden sein. Die Klebeverbindung kann durch einen Haftvermittler hergestellt werden. Der Haftvermittler kann beispielsweise Polyurethan umfassen oder daraus bestehen.

Die Abdeckung aus einem elektrogesponnenen Gewebe ist darüber hinaus äußerst dünn und flexibel, was die Flexibilität der Gitterstruktur unterstützt. Insbesondere hindert die Abdeckung die Gitterstruktur im Unterschied zu vorbekannten Abdeckungen, die aus Textilmaterialien hergestellt sind, kaum an der Komprimierung. Insgesamt lässt sich also die gesamte Abdeckvorrichtung auf einen erheblich kleineren Querschnittsdurchmesser komprimieren und so über kleine Katheter in besonders kleine Blutgefäße führen. Dies ist insbesondere für die Behandlung von Aneurysmen in zerebralen Blutgefäßen relevant, wozu sich die Erfindung besonders eignet.

Bevorzugt ist die Abdeckung durch ein Kunststoffmaterial, insbesondere durch ein Polymer, und vorzugsweise durch Polyurethan gebildet. Derartige Materialien sind besonders leicht und lassen sich gut in feinen Fäden durch ein elektrospinnendes Verfahren herstellen. Das Kunststoffmaterial ermöglicht es also einerseits eine besonders dünne und feinporige Abdeckung herzustellen. Andererseits weist das Kunststoffmaterial in sich bereits eine hohe Flexibilität auf, so dass eine hohe Komprimierbarkeit des medizinischen Sets erreicht wird. Alternativ kann die Abdeckung auch durch Polyethylen und oder Flurpolymeren gebildet sein.

Mit dem erfindungsgemäßen medizinischen Set sind daher auch Behandlungen in Blutgefäßen möglich, die mit bisherigen medizinischen Vorrichtungen, die eine Gitterstruktur und eine Abdeckung aufweisen, nicht erreicht werden können. Wegen der hohen Komprimierbarkeit der erfindungsgemäßen Vorrichtung treten bei der Zuführung durch einen Katheter sehr geringe Zuführkräfte auf. Das Material der Abdeckung kann zudem zur Reduktion der Zuführkräfte beitragen.

Insbesondere können die Zuführkräfte bei der Vorrichtung mit Abdeckung im Vergleich zur Zuführung der Gitterstruktur allein gleich oder kleiner sein.

Weiterhin kann durch die Abdeckvorrichtung, welche zweckdienlicherweise am Behandlungsort, also auf Höhe des Aneurysmas angeordnet ist, beim und nach dem Setzen der Coils ein Herauswandern der Coil aus dem Aneurysma verhindert werden, sodass die Gefahr eines durch die Coils verursachten Gefäßverschlusses ebenfalls zumindest stark reduziert und vorzugsweise ausgeschlossen werden kann. Speziell bei der Ausbildung der Abdeckvorrichtung als Flow Diverter, also als Dauerimplantat, kann auf ein Einsetzen der Coils in das Aneurysma verzichtet werden, da die Abdeckung das Aneurysma vorzugsweise dauerhaft, insbesondere fluiddynamisch abdeckt.

Ein weiterer Anwendungsbereich einer partiell abgedeckten Vorrichtung sind Fistulae, Dissektionen und weitere Malformationen, z.B. arteriovenöse Malformationen, in denen der Fluss an gezielten Stellen verlangsamt bzw. unterbunden werden soll, bzw. die Gefäßwandrekonstruktion durch eine feinmaschige Struktur zur Förderung der Zellproliferation begünstigt wird. In den offenen Bereichen bzw. in den offenen Zellen soll der Blutfluss ungehindert gewährleistet sein, damit Seitenäste normal durchblutet werden. Außerdem wird Fremdmaterial auf das Nötigste reduziert, zur Verbesserung der Biokompatibilität bzw. Reduzierung der möglichen Thrombogenität.

Um eine ausreichende Flexibilität der Abdeckung zu gewährleisten, ist diese vorzugsweise aus unregelmäßig netzartig angeordneten Fäden gebildet, die eine Fadendicke zwischen 0,1 µm und 3 µm, insbesondere zwischen 0,2 µm und 2 µm, insbesondere zwischen 0,5 µm und 1,5 µm, insbesondere zwischen 0,8 µm und 1,2 µm, aufweisen.

Besonders bevorzugt ist es, wenn das medizinische Set nach Art eines Stents zur Behandlung von Aneurysmen in arteriellen, insbesondere neurovaskulären, Blutgefäßen ausgebildet ist. Die Blutgefäße können vorzugsweise einen Querschnittsdurchmesser zwischen 1,5 mm und 5 mm, insbesondere zwischen 2 mm und 3 mm, aufweisen. Auch die Behandlung von Blutgefäßen mit einem Querschnittsdurchmesser von 4 mm bis 8 mm ist möglich. Derartige Querschnittsdurchmesser weisen beispielsweise Karotisarterien auf.

In einer weiteren Ausführungsform sind alle Stege der Innenzelle jeweils einer weiteren Innenzelle oder Randzelle zugeordnet. Weiterhin weisen die Randzellen jeweils wenigstens zwei Stege auf, die keiner weiteren Innenzelle oder Randzelle zugeordnet sind. Die Stege bilden somit in dieser Ausführungsform sowohl eine Begrenzung für eine Innenzelle als auch eine Begrenzung für ihre angrenzenden Randzellen auf.

Die Gitterstruktur kann grundsätzlich als einstückige Gitterstruktur ausgebildet sein. Das Drahtgeflecht der Gitterstruktur kann aus einem einzigen Draht bestehen, der an den Längsenden der Netzstruktur umgelenkt und zurückgeführt wird. Der Draht kann mit sich selbst verflochten werden, um die Netzstruktur zu bilden. Die Netzstruktur kann auch aus mehreren Drähten bestehen, die miteinander verflochten sind. Die mehreren Drähte können an einem axialen Längsende umgelenkt und zurückgeführt werden, während das gegenüberliegende axiale Längsende Drahtenden aufweisen kann, die offen sind. Es ist auch möglich, dass die miteinander verwebten Drähte offene Drahtenden an beiden axialen Längsenden aufweisen.. Der Draht kann ein röntgensichtbares Kernmaterial und ein Mantelmaterial aus einer Formgedächtnislegierung aufweisen. Insbesondere ist vorgesehen, dass das Volumenverhältnis zwischen dem Kernmaterial, vorzugsweise Platin, und dem Volumen des gesamten Verbunddrahts zwischen 20% und 40%, insbesondere zwischen 25% und 35% ist. Während eine geflochtene Gitterstruktur sich durch eine besonders hohe Flexibilität, insbesondere Biegeflexibilität, auszeichnet, weist eine einstückige Gitterstruktur eine vergleichsweise dünne Wandstärke auf, so dass die Gitterstruktur den Blutfluss innerhalb eines Blutgefäßes weniger stark beeinflusst. Die Gitterstruktur kann auch lasergeschnitten ausgebildet sein. Weiterhin kann die Gitterstruktur als eine geschlossene Struktur zum Zurückziehen in ein Katheter ausgebildet sein, mit zwischen vorzugsweise 6 und 12 Zellen entlang des Umfangs der Gitterstruktur. Die Zellen können eine asymmetrische Form aufweisen, d.h. die Zellen sind im Wesentlichen rautenförmig, also drachenförmig, können jedoch aufgrund variierender Stegbreiten und -längen von dieser Form abweichen. Die Rauten- bzw. Drachenform bleibt jedoch auch bei einer Abweichung im Wesentlichen erkennbar.

Die Gitterstruktur weist vorzugsweise einen Querschnittsdurchmesser zwischen 2,5 mm und 8 mm, insbesondere zwischen 4,5 mm und 6 mm, auf.

Gemäß einer bevorzugten Ausführungsvariante endet die Abdeckung an den Stegen der abdeckungsfreien Innenzellen derart, dass die Abdeckung nicht in die wenigstens eine Innenzelle hineinragt. Mit anderen Worten endet die Abdeckung somit im Wesentlichen bündig an den Stegen, sodass ein Rand der Abdeckung bevorzugt der Kontur der Stege folgt.

Hierdurch ist zum einen die Abdeckungsfreiheit der Randzellen gewährleistet und gleichzeitig durch das Anliegen des Randes der Abdeckung am Steg eine zuverlässige Befestigung, beispielsweise mittels der bereits erwähnten Klebeverbindung, an der Gitterstruktur sichergestellt.

Gemäß einer alternativen Ausführungsvariante überlappt die Abdeckung zumindest teilweise die Stege der abdeckungsfreien Innenzelle derart, dass die Abdeckung teilweise in die wenigstens eine abdeckungsfreie Innenzelle hineinragt. Mit anderen Worten folgt gemäß dieser alternativen Ausführungsform die Abdeckung gerade nicht der Kontur der Stege und ragt vielmehr über mindestens 20%, insbesondere über mindestens 30%, insbesondere über mindestens 40%, insbesondere über mindestens 50%, der Fläche der wenigstens einen Innenzelle in diese hinein.

In beiden Ausgestaltungsvarianten bleibet die Abdeckung jedoch stabil. Vorzugsweise ist die Abdeckung derart langzeitbeständig bzw. stabil, dass die Masse der Abdeckung bei einem Kontakt mit Blut oder einer physiologischen Ersatzflüssigkeit, insbesondere mit einer Natriumchlorid-Lösung oder einer Ringer-Lactat-Lösung bei einer Durchströmung von insbesondere 100ml bis 400ml pro Minute, über einen Zeitraum von wenigstens vier Stunden, insbesondere wenigstens 30 Tagen, um höchstens 5%, insbesondere um höchstens 3%, insbesondere um höchstens 1%, reduziert wird. Damit ist sichergestellt, dass die Wirkung der Beschichtung über einen ausreichend langen Zeitraum besteht.

Besonders bevorzugt ist es, wenn die Abdeckung derart langzeitbeständig bzw. stabil ist, dass die Masse der Abdeckung bei einem Kontakt mit Blut oder einer physiologischen Ersatzflüssigkeit, insbesondere mit einer Natriumchlorid-Lösung oder einer Ringer-Lactat-Lösung, über einen Zeitraum von wenigstens vier Stunden, insbesondere wenigstens 30 Tagen, vollständig erhalten bleibt. Ein solcher Zeitraum ermöglicht es beispielsweise, dass die medizinische Vorrichtung von einer Endothelzellenschicht überzogen wird, so dass eine Thrombenbildung auf natürliche Weise vermieden wird. Die antithrombogene Beschichtung überbrückt insofern den Zeitraum bis zur natürlichen Einheilung bzw. Einkapselung der medizinischen Vorrichtung in eine Neointimaschicht, insbesondere aus Endothelzellen, die sich um die Netzstrukturelemente bildet.

Die Nutzung einer physiologischen Ersatzflüssigkeit zum Test der Langzeitbeständigkeit der Abdeckung ermöglicht einen objektiven Vergleich. Ferner wird durch die Verwendung der Ersatzflüssigkeit, die dem menschlichen Blut vorzugsweise ähnlich ist, erreicht, dass daraus objektive Erfahrungswerte ermittelt werden können, die auf das Verhalten der Abdeckung im implantierten Zustand schließen lassen, wenn die Abdeckung dem menschlichen Blutfluss ausgesetzt ist. Daher werden als Ersatzflüssigkeiten vorzugsweise eine 0,9-prozentige Natriumchlorid-Lösung oder eine Ringer-Lactat-Lösung verwendet. Derartige Ersatzflüssigkeiten sind isotonisch und eignen sich gut als Indikator für das Verhalten der Abdeckung im implantierten Zustand.

Gemäß einer Ausführungsform sind mehrere, in Umfangsrichtung der Gitterstruktur unmittelbar benachbarte, Innenzellen, insbesondere alle Innenzellen, eines Innenzellenrings abdeckungsfrei. Hierdurch ist sichergestellt, dass benachbarte Seitengefäße und/oder Zellen entlang des Gefäßes weiterhin mit Blut und somit mit Nährstoffen versorgt werden können, während das Aneurysma zuverlässig durch die Abdeckung abgedeckt ist. Weiterhin ist hierdurch eine individuelle Abdeckwirkung/-funktion von Gitterstrukturen im Hinblick auf ein Anordnungsmuster von Aneurysmen entlang des Gefäßes ermöglicht, sodass beispielsweise lediglich an den Behandlungsorten an, an denen sich ein Aneurysma befindet, die Innenzellen mit der Abdeckung versehen sind und die übrigen Innenzellen des Innenzellenrings abdeckungsfrei sind.

Alternativ oder ergänzend sind mehrere, in Längsrichtung der Gitterstruktur unmittelbar benachbarte Innenzellen abdeckungsfrei. Hierdurch wird die bereits zuvor erwähnte Möglichkeit der individuellen Abdeckwirkung/-funktion der Abdeckvorrichtung vorteilhaft optimiert.

Gemäß einer Ausführungsform erstreckt sich die Abdeckung nur teilweise, insbesondere zu höchstens 50%, insbesondere zu höchstens 40%, insbesondere zu höchstens 30%, insbesondere zu höchstens 20%, über den Umfang der Gitterstruktur. Bevorzugt erstreckt sich somit die Abdeckung somit lediglich über den Behandlungsort, also beispielsweise über eine Öffnung des Aneurysmas. Durch diese Weiterbildung wird sichergestellt, dass das Aneurysma fluiddynamisch vollständig abgekoppelt ist bei der Verwendung der Gitterstruktur als Flow Diverter. Zum anderen ist hierdurch sichergestellt, dass insbesondere sich auf Höhe des Aneurysmas befindliche Zellen und/oder Seitengefäße durch die fehlende Abdeckung weiterhin mit Blut und somit mit Nährstoffen versorgt werden können.

In einer Ausführungsform weist die Abdeckung auf einer Fläche von 100.000 µm² mindestens 10 Poren auf, die eine Größe von mindestens 15 µm² aufweisen. Im Zuge der Herstellung der Abdeckung lässt sich die Mindestgröße der Poren insbesondere durch die Prozessdauer des Elektrospinnens einstellen. Diese Kombination aus einer bestimmten Mindestanzahl an Poren und einer Mindestgröße dieser Poren hat sich in der Praxis als besonders förderlich für eine ausreichende Blutdurchlässigkeit der Abdeckung bei gleichzeitig guter Abdeckwirkung gezeigt.

Ein nebengeordneter Aspekt der Erfindung betrifft ein Verfahren zur Herstellung eines medizinischen Sets, wobei sich hierbei vorzugsweise um das bereits vorstehend beschriebene medizinische Set handelt. Hierbei umfasst das Verfahren die nachfolgenden Schritte.

Zunächst wird die Gitterstruktur bereitgestellt. Anschließend wird hierauf die Abdeckung auf allen Innenzellen, speziell durch einen Elektrospinnprozess aufgebracht.

Daran anschließend wird wenigstens eine Innenzelle mittels eines Schneidwerkzeugs freigeschnitten, wobei das Schneidwerkzeug entlang der Stege der freizuschneidenden Innenzelle geführt wird. Die Innenzelle wird hierdurch vollständig abdeckungsfrei.

Bei dem Schneidwerkzeug kann es sich hierbei um ein mechanisches Schneidwerkzeug, beispielsweise um ein Skalpell handeln. Bevorzugt handelt es sich bei dem Schneidwerkzeug um ein Laser-Schneidwerkzeug, sodass die Abdeckung entlang der Stege mittels eines Laserstrahls entfernt wird, sodass die Innenzelle freigeschnitten wird. Alternativ kann es sich bei der Schneidvorrichtung auch um eine Vorrichtung handeln, die Abdeckungsfreiheit der wenigstens einen Innenzelle durch Ätzen sicherstellt.

Weiterhin kann ergänzend vorgesehen sein, dass die Kanten der Abdeckung an den abdeckungsfreien Innenzellen nachbearbeitet werden. Dies kann bevorzugt thermisch, beispielsweise mittels eines Lötkolbens erfolgen, indem in einem ersten Schritt bei einer ersten Temperatur eine Grobbearbeitung der Kanten erfolgt und anschließend bei einer, in Bezug auf die erste Temperatur, kleineren Temperatur eine Feinbearbeitung der Kanten erfolgt.

Ein weiterer nebengeordneter Aspekt der Erfindung betrifft ein medizinisches System mit einem medizinischen Set, wobei sich hierbei insbesondere um das bereits vorstehend beschriebene medizinische Set handelt, sodass das medizinische Set einen Hauptkatheter und eine durch den Hauptkatheter hindurch an einen Behandlungsort bewegbare Abdeckvorrichtung zum Abdecken eines Aneurysmas aufweist.

Die Abdeckvorrichtung ist hierbei mit einem Transportdraht verbunden oder verbindbar. Weiterhin weist die Abdeckvorrichtung eine selbstexpandierbare Gitterstruktur auf, die Stege umfasst, welche einstückig miteinander verbunden sind und Innenzellen sowie Randzellen begrenzen. Die Randzellen bilden an einem Längsende der Gitterstruktur einen geschlossenen Randzellenring entlang des Umfangs der Gitterstruktur. Der Randzellenring ist lediglich einseitig, also an der Seite, die in Richtung der Innenzellen orientiert ist, mit Innenzellen verbunden. Weiterhin weist das medizinisches System wenigstens ein Embolisationsmittel zur Platzierung im Aneurysma auf. Bevorzugt ist das Embolisationsmittel durch einen plastisch verformbaren Draht, insbesondere durch ein Coil oder durch eine Flüssigkeit, gebildet. Derartige Ausbildungen des Embolisationsmittels haben sich im Hinblick auf eine Behandlung von Aneurysmen als besonders geeignet erwiesen.

Im Fall, dass die Abdeckvorrichtung lösbar, also reversibel mit dem Transportdraht verbunden ist, dient die Abdeckvorrichtung besonders bevorzugt als Flow Diverter einer dauerhaften Implantation am Behandlungsort innerhalb des Blutgefäßes. Die reversible Anordnung der Abdeckvorrichtung an dem Transportdraht ist beispielsweise mittels einer lösbaren mechanischen Arretierung realisiert. Weiterhin weist die als Flow Diverter ausgebildete Abdeckungsvorrichtung in dieser Ausführungsform im implantierten und expandierten Zustand offene Längsenden auf, ist also beidendseitig offen ausgebildet. Die Abdeckvorrichtung ist somit im Wesentlichen zylinderförmig, also rohrartig, ausgebildet, sodass ein Blutfluss durch das Blutgefäß weiterhin ermöglicht ist. Somit ist eine Platzierung des Embolisationsmittels - sofern erforderlich - sowohl im Rahmen der Implantation der Abdeckvorrichtung als auch zu einem späteren, sich an die Implantation der Abdeckvorrichtung anschließenden, Zeitpunkt ermöglicht.

In einer Ausführungsform und bevorzugt weist das medizinisches System weiterhin einen Zusatzkatheter zur Zuführung des Embolisationsmittels in das Aneurysma auf, wobei der Zusatzkatheter vom Hauptkatheter unabhängig und/oder gegenüber dem Hauptkatheter relativbeweglich ist.

Die im Hinblick auf das medizinische Set aufgeführten Vorteile und bevorzugten Ausgestaltungen sind sinngemäß auf das Verfahren sowie auf das medizinische Set zu übertragen und umgekehrt. Alle in Bezug auf das medizinische Set und in Bezug auf das medizinische System aufgeführten Dimensionsangaben gelten für einen expandierten Zustand der Gitterstruktur, sofern nicht etwas Anderes angegeben ist.

Ausführungsbeispiele der Erfindung werden nachfolgend anhand der beigefügten, schematischen Zeichnungen näher erläutert. Diese zeigen in teilweise stark vereinfachter Darstellung:
- Fig. 1: eine Seitenansicht einer Ausführungsform des erfindungsgemäßen medizinischen Sets,
- Fig. 2: eine Nahaufnahme einer bevorzugten Ausführungsform einer Abdeckungsvorrichtung eines erfindungsgemäßen medizinischen Sets gemäß einer bevorzugten Ausführungsform,
- Fig. 3: eine Rasterelektronenmikroskopaufnahme einer Abdeckung einer Abdeckvorrichtung eines erfindungsgemäßen medizinischen Sets gemäß einer bevorzugten Ausführungsform,
- Fig. 4: eine Seitenansicht einer Ausführungsform des erfindungsgemäßen medizinischen Systems, bei einer temporären Implantation der Abdeckvorrichtung an einem Behandlungsort sowie
- Fig. 5: eine Seitenansicht einer bevorzugten Ausführungsform des erfindungsgemäßen medizinischen Systems, bei einer dauerhaften Implantation der Abdeckvorrichtung an einem Behandlungsort.

In den Figuren sind gleichwirkende Teile stets mit den gleichen Bezugszeichen dargestellt.

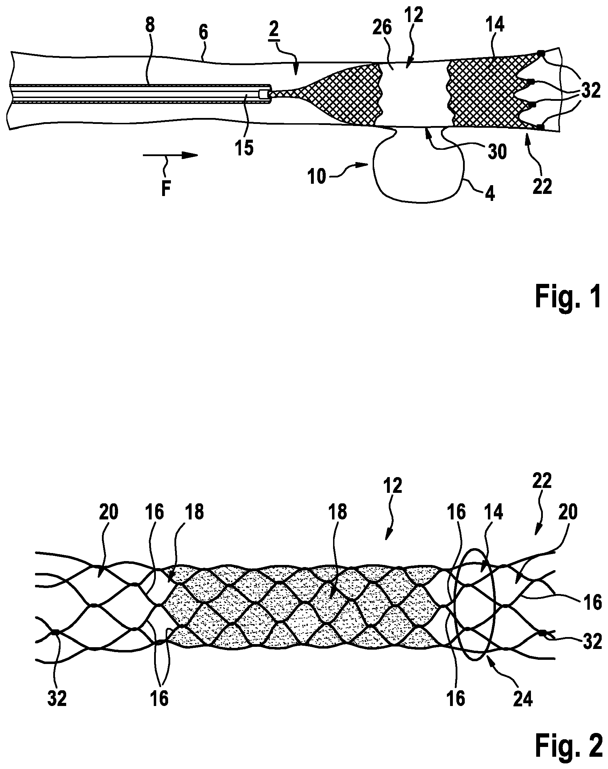

Das in Fig. 1 schematisch dargestellte medizinische Set 2 dient zur Behandlung von Aneurysmen 4 und ist in Fig. 1 in einem innerhalb eines Gefäßes 6 angeordneten Zustand gezeigt.

Das medizinische Set 2 weist einen Hauptkatheter 8 sowie eine durch den Hauptkatheter 8 hindurch an einen Behandlungsort 10 bewegbare Abdeckvorrichtung 12 auf. Bei den Behandlungsort 10 handelt es sich vorzugsweise um den Ort entlang des Gefäßes 6, an dem das Aneurysma 4 ausgebildet ist. Die Abdeckvorrichtung 12 dient einem temporären oder dauerhaften Abdecken des Aneurysmas 4, wobei die Abdeckvorrichtung 12 eine selbstexpandierbare Gitterstruktur 14 aus Stegen 16 (vgl. Fig. 2) umfasst. Die Gitterstruktur 14 ist im Hinblick auf ihre Selbstexpandierbarkeit bevorzugt aus einem Formgedächtnismaterial gefertigt.

Weiterhin ist die Abdeckvorrichtung 12 dauerhaft oder lösbar mechanisch mit einem innerhalb des Hauptkatheters 8 verschiebbaren Transportdraht 15 verbunden. Durch diesen Transportdraht 15 ist die Gitterstruktur 14 und somit die Abdeckvorrichtung 12 in einem nicht expandierten Zustand durch den Hauptkatheter 8 schiebbar und zurückziehbar

Im expandierten Zustand ist die Gitterstruktur 14 längsaxial, also in und entgegen einer Fließrichtung F blutdurchströmbar.

Die Gitterstruktur 14 ist insbesondere derart innerhalb des Gefäßes 6 angeordnet, dass die Abdeckung 26 auf einer Höhe mit einer Öffnung 30 des Aneurysmas 4 platziert ist, sodass dieses von der Abdeckung 26 abgedeckt wird, wobei gleichzeitig ein Blutfluss durch das Gefäß 6 nicht gestoppt wird. Die Abdeckung 26 ist somit im Ausführungsbeispiel gemäß Fig. 1 im Wesentlichen rohrartig, insbesondere nach Art eines Hohlzylinders, ausgebildet.

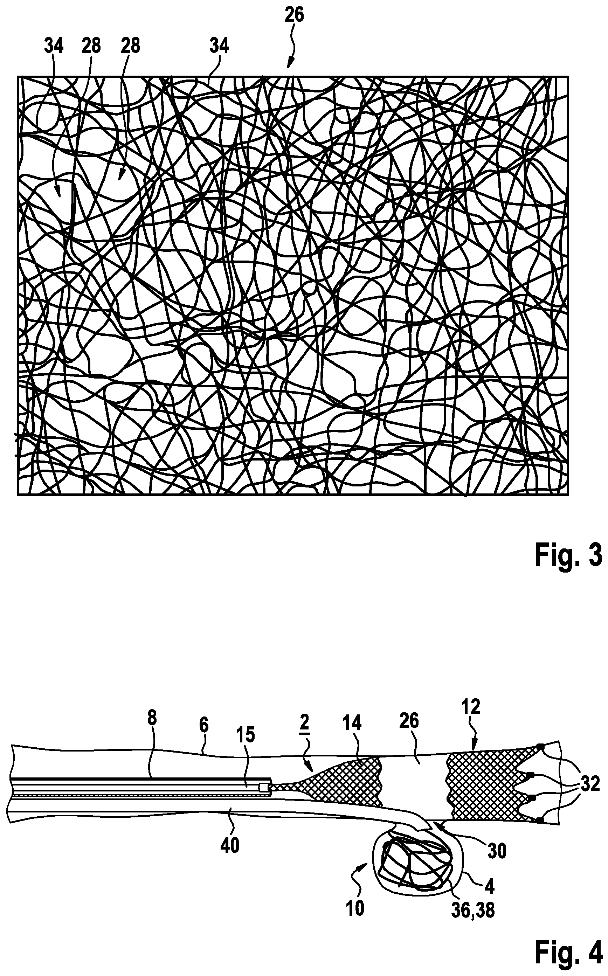

Die Abdeckung 26 ist hierbei vorzugsweise porös mit großen Poren 28 (vgl. Fig. 3) und blutdurchlässig ausgebildet, sodass eine Nährstoffversorgung, der von der Abdeckung 26 abgedeckten Zellen weiterhin gewährleistet ist. Alternativ kann die Abdeckung 26 jedoch auch porös und blutundurchlässig ausgebildet sein.

Die Abdeckung 26 dient hierbei, insbesondere bei einem Platzieren eines Embolisationsmittels 36 (vgl. Fig. 4) innerhalb des Aneurysmas 4, dazu, dass das Embolisationsmittel 40 nach dem Platzieren nicht aus dem Aneurysma 4 entweichen kann bis das Blut innerhalb des Aneurysmas 4 durch das Embolisationsmittel 36 geronnen und somit das Aneurysma 4 zuverlässig verschlossen ist.

Besonders bevorzugt ist die Abdeckung 26 als eine elektrogesponnene Abdeckung 26 ausgebildet.

Wie in Fig. 1 gut zu sehen ist, sind zudem bei dem medizinischen Set 2 und speziell bei der Gitterstruktur 14 Röntgenmarker 32 vorgesehen. Die Röntgenmarker 32 sind vorzugsweise an Zellenspitzen von Randzellen 20 (vgl. Fig. 2) der Gitterstruktur 14 angeordnet. Konkret können die Röntgenmarker 56 als röntgensichtbare Hülsen, beispielsweise aus Platin oder Gold, gebildet sein, die auf die Zellenspitzen der Randzellen 20 aufgecrimpt sind.

In Fig. 2 ist eine Nahaufnahme einer bevorzugten Ausführungsform der erfindungsgemäßen Abdeckungsvorrichtung 12 dargestellt. Die Stege 16 sind einstückig, also monolithisch, miteinander verbunden und begrenzen Innenzellen 18 sowie Randzellen 20. Vorzugsweise sind alle Zellen, insbesondere Innenzellen 18 und Randzellen 20 jeweils durch vier Stege begrenzt, die gemeinsam eine Rautenform ergeben. Diese allseitig umschlossenen Zellen werden als geschlossene Zellen bezeichnet. Die Randzellen 20 bilden an einem Längsende 22 der Gitterstruktur 14 einen geschlossenen Randzellenring 24 (drei Randzellen 20 des Randzellenrings 24 sind eingekreist dargestellt) in Umfangsrichtung, der nur einseitig mit Innenzellen 18 verbunden ist.

Weiterhin ist die Gitterstruktur 14 mit der Abdeckung 26 aus einem Gewebe versehen, das unregelmäßig große Poren 28 (vgl. Fig. 3) aufweist. Wenigstens eine Innenzelle 18, in Fig. 1 (in der Bildebene am rechten Rand der Gitterstruktur 14) drei Innenzellen 18, sind abdeckungsfrei ausgebildet.

Die Gestaltung der Abdeckung 26 ist in der Rasterelektronenmikroskopaufnahme gemäß Fig. 3 gut erkennbar. Darin ist zu sehen, dass die Abdeckung 26 mehrere unregelmäßig große Poren 28 aufweist, die jeweils durch Fäden 34 begrenzt sind. Durch den Elektrospinnprozess werden mehrere Fäden 34 gebildet, die unregelmäßig zueinander ausgerichtet sind. Dabei bilden sich die Poren 28. Erkennbar ist in Fig. 3 auch, dass die Poren 28 eine vergleichsweise kleine Porengröße aufweisen, wobei einige Poren 28 jedoch ausreichend groß sind, um beispielsweise eine Blutdurchlässigkeit zu gewährleisten. Konkret sind in Fig. 3 vier Poren 28 grafisch hervorgehoben, die eine Größe von mehr als 30 µm² aufweisen. Die Dichte der Poren 28 mit einer Größe von mehr als 30 µm² lässt erkennen, dass die Abdeckung auf einer Fläche von 100.000 µm² wenigstens 10 derartiger Poren 28 aufweist.

In Fig. 3 ist jeweils auch erkennbar, dass sich die Fäden 34 der Abdeckung 26 mehrfach kreuzen. Eine Besonderheit des Elektrospinnverfahrens ist es jedoch, dass bei der Abdeckung 26 Stellen vorliegen, an welchen sich ausschließlich, d.h. nicht mehr als, zwei Fäden 34 überkreuzen. Daraus ist ersichtlich, dass die Abdeckung 26 insgesamt eine sehr dünne Wandstärke aufweist und daher hochflexibel ist.

Die hohe Flexibilität der Abdeckung 26 in Kombination mit der hohen Flexibilität der Gitterstruktur 14 führt dazu, dass eine Abdeckvorrichtung 12 bereitgestellt werden kann, die durch sehr kleine Zuführkatheter in ein (Blut-)Gefäß 6 eingeführt werden kann. Insbesondere können Zuführkatheter eingesetzt werden, die eine Größe von 6 French, insbesondere höchstens 5 French, insbesondere höchstens 4 French, insbesondere höchstens 3 French, insbesondere höchstens 2 French, aufweisen. Konkret kann die Abdeckvorrichtung nach den hier beschriebenen Ausführungsbeispielen bei Kathetern eingesetzt werden, die einen Innendurchmesser von höchstens 1,6 mm, insbesondere höchstens 1,0 mm, insbesondere höchstens 0,7 mm, insbesondere höchstens 0,4 mm aufweisen.

Die Schichtdicke der Abdeckung 26 beträgt in besonders bevorzugten Varianten höchstens 10 µm, insbesondere höchstens 8 µm, insbesondere höchstens 6 µm, insbesondere höchstens 4 µm. Dabei überkreuzen sich höchstens 4, insbesondere höchstens 3, insbesondere höchstens 2, Fäden 34. Generell sind innerhalb der elektrogesponnenen Struktur der Abdeckung 26 Kreuzungspunkte vorgesehen, in welchen sich nur 2 Fäden 34 überkreuzen. Die Gitterstruktur 10 weist vorzugsweise einen Querschnittsdurchmesser zwischen 2,5 mm und 8 mm, insbesondere zwischen 4,5 mm und 6 mm, auf.

Fig. 4 zeigt ein schematisch dargestelltes Ausführungsbeispiel eines erfindungsgemäßen medizinischen Systems bei einer temporären Implantation der Abdeckvorrichtung (12) an einem Behandlungsort 10.

Das medizinische System weist hierbei das bereits vorstehend erwähnte medizinische Set 2 mit dem Hauptkatheter 8 sowie der Abdeckvorrichtung 12, die die Gitterstruktur 14 und die Abdeckung 26 aufweist, auf. Die Ausgestaltung der Abdeckung 26 entspricht hierbei der bereits vorstehend erwähnten Ausführungsform gemäß Fig. 1.

Weiterhin weist das medizinische System ein Embolisationsmittel 36 auf, welches beispielsweise durch einen plastisch verformbaren Draht 38 oder durch eine Flüssigkeit gebildet ist. Das Embolisationsmittel 36 wird mittels eines Zusatzkatheters 40, welcher ebenfalls Teil des medizinischen Systems ist, innerhalb des Aneurysmas 4 platziert.

Zum Platzieren des Embolisationsmittels 36 ist der Zusatzkatheter 40 gemäß der dargestellten Ausführungsform des medizinischen Systems im Wesentlichen parallel, also neben dem medizinischen Set 2 innerhalb des Gefäßes 6 angeordnet. Hierbei wird dann der Zusatzkatheter 40 und speziell eine Spitze des Zusatzkatethers 40 zwischen einer Gefäßwand und der Abdeckung 26 in das Aneurysma 4 "geschoben", um dort das Embolisationsmittel 36 in Form des plastisch verformbaren Drahtes 38 (auch als "Coil" bezeichnet) zu platzieren. Die Abdeckung 26 verhindert währenddessen und - bei einer Dauerimplantation - auch anschließend, dass das Embolisationsmittel 36 beispielsweise aufgrund der Blutströmung aus dem Aneurysma 4 heraustritt.

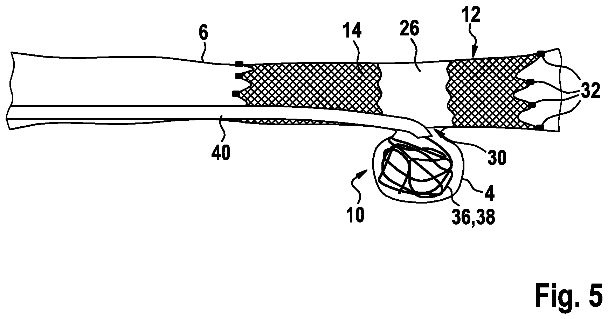

In Fig. 5 ist ein schematisch dargestelltes Ausführungsbeispiel eines erfindungsgemäßen medizinischen Systems bei einer dauerhaften Implantation der Abdeckvorrichtung (12) an einem Behandlungsort 10 gezeigt.

Bezüglich der strukturellen Merkmale entspricht das medizinische Set sowie die Abdeckvorrichtung 12 im Wesentlichen dem bereits vorstehend in Fig. 4 beschriebenen medizinischen Set. Die Abdeckvorrichtung 12 ist in Fig. 5 in einem bereits in dem Gefäß 6 implantierten und expandierten Zustand gezeigt. D.h. die Abdeckvorrichtung 12 wurde bereits mittels des Transportdrahtes 15 an den Behandlungsort 10 verbracht und dort von dem Transportdraht 15 abgelöst.

Die Abdeckvorrichtung 12 gemäß Fig. 5 weist jedoch jeweils offene Längsenden auf und ist somit im Wesentlichen zylinderförmig, also rohrartig ausgebildet, um einen Blutfluss durch das Gefäß nicht zu beeinflussen. Die Abdeckvorrichtung 12 im Ausführungsbeispiel gemäß Fig. 5 dient somit als Flow Diverter.

### Bezugszeichenliste

- 2: medizinisches Set
- 4: Aneurysma
- 6: Gefäß
- 8: Hauptkatheter
- 10: Behandlungsort
- 12: Abdeckvorrichtung
- 14: Gitterstruktur
- 15: Transportdraht
- 16: Steg
- 18: Innenzelle
- 20: Randzelle
- 22: Längsende
- 24: Randzellenring
- 26: Abdeckung
- 28: Pore
- 30: Öffnung des Aneurysmas
- 32: Röntgenmarker
- 34: Faden
- 36: Embolisationsmittel
- 38: Draht
- 40: Zusatzkatheter

- F: Fließrichtung

## Patentansprüche

1. Medizinisches Set (2) zur Behandlung von Aneurysmen (4) mit einem Hauptkatheter (8), einer durch den Hauptkatheter (8) hindurch an einen Behandlungsort (10) bewegbaren Abdeckvorrichtung (12) zum Abdecken eines Aneurysmas (4), wobei die Abdeckvorrichtung (12) mit einem Transportdraht (15) verbunden oder verbindbar ist und eine selbstexpandierbare Gitterstruktur (14) aus Stegen (16) umfasst, die einstückig miteinander verbunden sind und Innenzellen (18) sowie Randzellen (20) begrenzen, wobei die Randzellen (20) an einem Längsende (22) der Gitterstruktur (14) einen geschlossenen Randzellenring (24) bilden, der nur einseitig mit Innenzellen (18) verbunden ist,
**dadurch gekennzeichnet, dass**
die Gitterstruktur (14) mit einer Abdeckung (26) aus einem Gewebe versehen ist, das unregelmäßig große Poren (28) aufweist, wobei wenigstens eine Innenzelle (18) der Gitterstruktur (14) zumindest teilweise, insbesondere größtenteils, abdeckungsfrei ist.

2. Medizinisches Set (2) nach Anspruch 1,
**dadurch gekennzeichnet, dass**
die Abdeckung (26) aus einem elektrogesponnenen Gewebe gebildet ist.

3. Medizinisches Set (2) nach Anspruch 1 oder 2,
**dadurch gekennzeichnet, dass**
die Abdeckvorrichtung (12) reversibel mit dem Transportdraht (15) verbindbar ist und im expandierten Zustand beidendseitig offen und blutdurchströmbar ist.

4. Medizinisches Set (2) nach einem der vorhergehenden Ansprüche,
**dadurch gekennzeichnet, dass**
alle Stege (16) der Innenzellen (18) jeweils einer weiteren Innenzelle (18) oder Randzelle (20) zugeordnet sind und die Randzellen (20) jeweils wenigstens zwei Stege (16) aufweisen, die keiner weiteren Innenzelle (18) oder Randzelle (20) zugeordnet sind.

5. Medizinisches Set (2) nach einem der vorhergehenden Ansprüche,
**dadurch gekennzeichnet, dass**
die Abdeckung (26) an den Stegen (16) der abdeckungsfreien Innenzelle (18) endet, derart, dass die Abdeckung (26) nicht in die abdeckungsfreie Innenzelle (18) hineinragt.

6. Medizinisches Set (2) nach einem der Ansprüche 1 bis 4,
**dadurch gekennzeichnet, dass**
die Abdeckung (26) die Stege (16) der abdeckungsfreien Innenzelle (18) zumindest teilweise überlappt, so dass die Abdeckung (26) teilweise in die abdeckungsfreie Innenzelle (18) hineinragt.

7. Medizinisches Set (2) nach einem der vorhergehenden Ansprüche,
**dadurch gekennzeichnet, dass**
mehrere, in Umfangsrichtung der Gitterstruktur (14) unmittelbar benachbarte Innenzellen (18), insbesondere alle Innenzellen (18) eines Innenzellenrings, abdeckungsfrei sind.

8. Medizinisches Set (2) nach einem der vorhergehenden Ansprüche,
**dadurch gekennzeichnet, dass**
mehrere, in Längsrichtung der Gitterstruktur (14) unmittelbar benachbarte Innenzellen (18) abdeckungsfrei sind.

9. Medizinisches Set (2) nach einem der vorhergehenden Ansprüche,
**dadurch gekennzeichnet, dass**
sich die Abdeckung (26) nur teilweise, insbesondere zu höchstens 50%, insbesondere zu höchstens 40%, insbesondere zu höchstens 30%, insbesondere zu höchstens 20% über den Umfang der Gitterstruktur (14) erstreckt.

10. Medizinisches Set nach einem der vorhergehenden Ansprüche,
**dadurch gekennzeichnet, dass**
die Abdeckung (26) auf einer Fläche von 100.000 µm² mindestens 10 Poren (28) umfasst, die eine Größe von mindestens 15 µm² aufweisen.

11. Verfahren zur Herstellung eines medizinischen Sets (2) nach einem der vorhergehenden Ansprüche, wobei das Verfahren die folgenden Schritte aufweist:
a. Bereitstellen der Gitterstruktur (14);
b. Aufbringen der Abdeckung (26) auf alle Innenzellen (18) durch einen Elektrospinnprozess;
c. Freischneiden wenigstens einer Innenzelle (18) mittels eines Schneidwerkzeugs, wobei das Schneidwerkzeug entlang der Stege (16) der freizuschneidenden Innenzelle (18) geführt wird, so dass die Innenzelle (18) zumindest teilweise, insbesondere größtenteils abdeckungfrei wird.

12. Medizinisches System mit einem medizinischen Set (2) nach einem der Ansprüche 1 bis 9 und mit wenigstens einem Embolisationsmittel (36) zur Platzierung im Aneurysma (4).

13. Medizinisches System nach Anspruch 12,
**dadurch gekennzeichnet, dass**
die Abdeckungsvorrichtung (12) mit dem Transportdraht (15) lösbar verbindbar ist, zu einer dauerhaften Implantation der Abdeckungsvorrichtung (12) am Behandlungsort (10), wobei die Abdeckungsvorrichtung (12) im expandierten Zustand offene Längsenden aufweist.

14. Medizinisches System nach Anspruch 12,
**dadurch gekennzeichnet, dass**
die Abdeckungsvorrichtung (12) mit dem Transportdraht (15) unlösbar verbunden ist, zu einer temporären Implantation der Abdeckungsvorrichtung (12) am Behandlungsort (10).

15. Medizinisches System nach einem der Ansprüche 12 bis 14,
**dadurch gekennzeichnet, dass**
weiterhin ein Zusatzkatheter (40) zur Zuführung des Embolisationsmittels (36) in das Aneurysma vorgesehen ist, wobei der Zusatzkatheter (40) vom Hauptkatheter (8) unabhängig und/oder gegenüber dem Hauptkatheter (8) relativbeweglich ist.

## Claims

1. A medical set (2) for treating aneurysms (4), with a main catheter (8), a covering device (12) movable through the main catheter (8) to a treatment site (10) in order to cover an aneurysm (4), wherein the covering device (12) is connected to connectable to a transport wire (15) and comprises a self-expandable mesh structure (14) formed by struts (16) which are connected to one another in one piece and delimit inner cells (18) and edge cells (20), wherein, at a longitudinal end (22) of the mesh structure (14), the edge cells (20) form a closed edge cell ring (24) which is connected to the inner cells (18) on only one side,
**characterized in that**
the mesh structure (14) is provided with a covering (26) formed from a fabric which has pores (28) of irregular sizes, wherein at least one inner cell (18) of the mesh structure (14) is at least partially, in particular mainly, free of the covering.

2. The medical set (2) as claimed in claim 1,
**characterized in that**
the covering (26) is formed from an electrospun fabric.

3. The medical set (2) as claimed in claim 1 or claim 2,
**characterized in that**
the covering device (12) can be reversibly connected to the transport wire (15) and, in the expanded state, is open at both ends and permeable to blood.

4. The medical set (2) as claimed in any of the preceding claims,
**characterized in that**
all of the struts (16) of the inner cells (18) are respectively associated with a further inner cell (18) or edge cell (20), and the edge cells (20) respectively have at least two struts (16) which are not associated with any other inner cells (18) or edge cells (20).

5. The medical set (2) as claimed in any of the preceding claims,
**characterized in that**
the covering (26) ends at the struts (16) of the inner cells (18) which are free of the covering in a manner such that the covering (26) does not protrude into the inner cell (18) which is free of the covering.

6. The medical set (2) as claimed in any one of claims 1 to 4,
**characterized in that**
the covering (26) at least partially overlaps the struts (16) of the inner cell (18) which is free of the covering, so that part of the covering (26) protrudes into the inner cell (18) which is free of the covering.

7. The medical set (2) as claimed in any of the preceding claims,
**characterized in that**
a plurality of inner cells (18) which are immediately adjacent in the circumferential direction of the mesh structure (14), in particular all of the inner cells (18) of an inner cell ring, are free of the covering.

8. The medical set (2) as claimed in any of the preceding claims,
**characterized in that**
a plurality of inner cells (18) which are immediately adjacent in the longitudinal direction of the mesh structure (14) are free of the covering.

9. The medical set (2) as claimed in any of the preceding claims,
**characterized in that**
the covering (26) extends only partially over the circumference of the mesh structure (14), in particular by at most 50%, in particular by at most 40%, in particular by at most 30%, in particular by at most 20%.

10. The medical set as claimed in any of the preceding claims,
**characterized in that**
the covering (26) comprises at least 10 pores (28) with a size of at least 15 µm² over an area of 100000 µm².

11. A method for manufacturing a medical set (2) as claimed in any of the preceding claims, wherein the method has the following steps:
a. providing the mesh structure (14);
b. applying the covering (26) to all of the inner cells (18) using an electrospinning process;
c. cutting at least one inner cell (18) free by means of a cutting tool, wherein the cutting tool is guided along the struts (16) of the inner cells (18) to be cut free so that the inner cell (18) is at least partially, in particular mainly, free of the covering.

12. A medical system with a medical set (2) as claimed in any of claims 1 to 9 and with at least one embolization means (36) for placement in the aneurysm (4).

13. The medical system as claimed in claim 12,
**characterized in that**
the covering device (12) is detachably connectable to the transport wire (15) for a permanent implantation of the covering device (12) at the treatment site (10), wherein in the expanded state, the covering device (12) has open longitudinal ends (22).

14. The medical system as claimed in claim 12,
**characterized in that**
the covering device (12) is non-detachably connected to the transport wire (15) for a temporary implantation of the covering device (12) at the treatment site (10).

15. The medical system as claimed in one of claims 12 to 14,
**characterized in that**
in addition, an additional catheter (40) is provided for delivering the embolization means (36) into the aneurysm, wherein the additional catheter (40) is relatively movable with respect to the main catheter (8) independently of and/or in relation to the main catheter (8).

## Revendications

1. Ensemble médical (2) pour le traitement d'anévrismes (4) avec un cathéter principal (8), un dispositif de recouvrement (12) pouvant être déplacé à travers le cathéter principal (8) jusqu'à un site de traitement (10) pour recouvrir un anévrisme (4), le dispositif de recouvrement (12) est relié ou reliable à un fil de transport (15) et comprenant une structure en treillis auto-expansible (14) composée de bandes (16) reliées d'un seul tenant les unes aux autres et délimitant les cellules internes (18) et les cellules périphériques (20), les cellules périphériques (20) formant à une extrémité longitudinale (22) de la structure en treillis (14) un anneau de cellules périphériques fermé (24) qui n'est relié que par un côté aux cellules intérieures (18),
**caractérisé en ce que** la structure en treillis (14) est pourvue d'un couvercle (26) formée d'un tissu qui présente des pores grossiers irréguliers (28), au moins une cellule intérieure (18) de la structure en treillis (14) étant au moins partiellement, en particulier majoritairement, exempte de couvercle.

2. Ensemble médical (2) selon la revendication 1, **caractérisé en ce que** le couvercle (26) est composé d'un tissu électrofilé.

3. Ensemble médical (2) selon la revendication 1 ou 2, **caractérisé en ce que** le dispositif de recouvrement (12) est reliable de manière réversible au fil de transport (15) et est ouvert des deux côtés à l'état déployé et est perméable au sang.

4. Ensemble médical (2) selon l'une quelconque des revendications précédentes,
**caractérisé en ce que** toutes les bandes (16) des cellules intérieures (18) sont associées chacune à une autre cellule intérieure (18) ou cellule périphériques (20) et que les cellules périphériques (20) présentent chacune au moins deux bandes (16) qui ne sont associées à aucune autre cellule intérieure (18) ou cellule périphériques (20).

5. Ensemble médical (2) selon l'une quelconque des revendications précédentes,
**caractérisé en ce que** le couvercle (26) se termine sur les bandes (16) de la cellule intérieure (18) sans couvercle, de telle sorte que le couvercle (26) ne dépasse pas dans la cellule intérieure (18) sans couvercle.

6. Ensemble médical (2) selon l'une des revendications 1 à 4,
**caractérisé en ce que** le couvercle (26) recouvre au moins partiellement les bandes (16) de la cellule intérieure (18) sans couvercle, de sorte que le couvercle (26) pénètre partiellement dans la cellule intérieure (18) sans couvercle.

7. Ensemble médical (2) selon l'une quelconque des revendications précédentes,
**caractérisé en ce que** plusieurs cellules intérieures (18) directement voisines dans la direction circonférentielle de la structure en treillis (14), en particulier toutes les cellules intérieures (18) d'un anneau cellulaire intérieur, sont exemptes de couvercle.

8. Ensemble médical (2) selon l'une quelconque des revendications précédentes,
**caractérisé en ce que** plusieurs cellules intérieures (18) directement voisines dans la direction longitudinale de la structure en treillis (14) sont exemptes de couvercle.

9. Ensemble médical (2) selon l'une quelconque des revendications précédentes,
**caractérisé en ce que** le couvercle (26) s'étend seulement partiellement, en particulier jusqu'à un maximum de 50 %, en particulier jusqu'à un maximum de 40 %, en particulier jusqu'à un maximum de 30 %, en particulier jusqu'à un maximum de 20 %, sur la circonférence de la structure en treillis (14).

10. Ensemble médical selon l'une quelconque des revendications précédentes,
**caractérisé en ce que** le couvercle (26), sur une surface de 100 000 µm², comprend au moins 10 pores (28), qui ont une taille d'au moins 15 µm².

11. Procédé de fabrication d'un ensemble médical (2) selon l'une quelconque des revendications précédentes, le procédé comprenant les étapes consistant à :
a. mettre à disposition la structure en treillis (14) ;
b. appliquer le couvercle (26) sur toutes les cellules intérieures (18) au moyen d'un procédé d'électrofilage ;
c. découpe d'au moins une cellule intérieure (18) au moyen d'un outil de découpe, l'outil de découpe étant guidé le long des bandes (16) de la cellule intérieure (18) à découper, de sorte que la cellule intérieure (18) est au moins partiellement, en particulier majoritairement, exempte de couvercle.

12. Système médical avec un ensemble médical (2) selon l'une quelconque des revendications 1 à 9 et avec au moins un agent d'embolisation (36) destiné à être placé dans l'anévrisme (4).

13. Système médical selon la revendication 12,
**caractérisé en ce que** le dispositif de recouvrement (12) est reliable de manière amovible au fil de transport (15) pour une implantation permanente du dispositif de recouvrement (12) sur le site de traitement (10), le dispositif de recouvrement (12) présentant à l'état déployé des extrémités longitudinales ouvertes.

14. Système médical selon la revendication 12,
**caractérisé en ce que** le dispositif de recouvrement (12) est relié de manière inséparable au fil de transport (15), pour une implantation temporaire du dispositif de recouvrement (12) sur le site de traitement (10).

15. Système médical selon l'une quelconque des revendications 12 à 14,
**caractérisé en ce qu'**il est en outre prévu un cathéter supplémentaire (40) pour introduire l'agent d'embolisation (36) dans l'anévrisme, le cathéter supplémentaire (40) étant indépendant du cathéter principal (8) et/ou mobile par rapport au cathéter principal (8).
